(19) Europäisches Patentamt
European Patent Office
Office européen des brevets

(11) Veröffentlichungsnummer : **0 467 841 A2**

# (12) EUROPÄISCHE PATENTANMELDUNG

(21) Anmeldenummer : **91810545.3**

(22) Anmeldetag : **09.07.91**

(51) Int. Cl.$^5$ : **C07C 69/76,** C07C 67/04, C07D 309/10, C07D 307/12, C08F 12/22, G03F 7/039

(30) Priorität : **18.07.90 CH 2385/90**

(43) Veröffentlichungstag der Anmeldung : **22.01.92 Patentblatt 92/04**

(84) Benannte Vertragsstaaten : **AT BE CH DE FR GB IT LI NL SE**

(71) Anmelder : **CIBA-GEIGY AG**
**Klybeckstrasse 141**
**CH-4002 Basel (CH)**

(72) Erfinder : **Steinmann, Alfred, Dr.**
**Les Russilles**
**CH-1724 Praroman (CH)**

## (54) Einen olefinisch ungesättigten Substituenten aufweisende Benzoesäureester.

(57) Aus einen olefinisch ungesättigten Substituenten aufweisenden Benzoesäureestern der Formel I

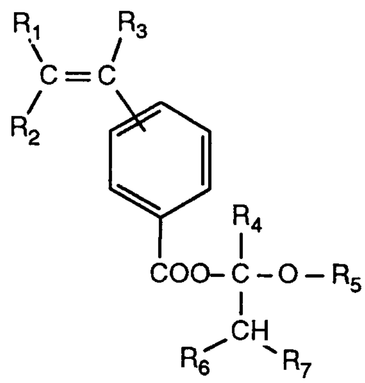

(I),

worin $R_1$ und $R_2$ unabhängig voneinander je ein Wasserstoffatom, $C_1$-$C_4$-Alkyl oder Phenyl, $R_3$ ein Wasserstoffatom, Methyl oder ein Halogenatom, $R_4$ ein Wasserstoffatom oder Methyl und $R_5$ ein $C_1$-$C_4$-Alkyl oder $C_6$-$C_{12}$-Aryl und $R_6$ und $R_7$ unabhängig voneinander je ein Wassterstoffatom, ein $C_1$-$C_4$-Alkyl oder ein $C_6$-$C_{12}$-Aryl bedeuten, wobei $R_6$ und $R_7$ zusammen auch einen unsubstituierten oder einen $C_1$-$C_4$-alkyl-, $C_1$-$C_4$-alkoxy-, $C_6$-$C_{12}$-aryl- oder $C_6$-$C_{12}$-aryloxysubstituierten Ethylen-, Propylen- oder Butylenrest bedeuten können, lassen sich gegebenenfalls in Gegenwart von anderen olefinisch ungesättigten Monomeren Polymere herstellen, die für positiv arbeitende Photoresists verwendet werden können.

EP 0 467 841 A2

Gegenstand vorliegender Erfindung sind neue, einen olefinisch ungesättigten Substituenten aufweisende Benzoesäureester, die unter Verwendung der erfindungsgemässen Benzoesäureester hergestellten Polymeren und positiv arbeitende, strahlungsempfindliche Mischungen, enthaltend die neuen Polymeren und eine bei aktinischer Bestrahlung säurebildende Substanz.

Positiv arbeitende, strahlungsempfindliche Mischungen und deren Verwendung als Photoresist sind bekannt. Neben einer hohen Empfindlichkeit im UV werden von solchen Photoresists eine einfache Entwickelbarkeit und gute Aetzresistenz gefordert.

Beispielsweise werden im US-Patent 3,779,778 positiv arbeitende Photoresists beschrieben, enthaltend eine wasserlösliche organische Verbindung, die bestimmte säurespaltbare Bindungen aufweist, und eine bei aktinischer Bestrahlung säurebildende Verbindung. Diese Photoresists können nur in relativ stark alkalischen wässrigen Lösungen entwickelt werden, und sie sind wenig temperaturstabil.

Die aus der EP-A-0 254 853 bekannten, positiv arbeitenden Photoresists ergeben nach der Belichtung und Entwicklung Bilder mit einer hohen thermischen Stabilität, doch sind sie relativ wenig lichtempfindlich und nur in stark alkalischen, wässrigen Lösungen entwickelbar.

Es wurde nun gefunden, dass man Polymere aus bestimmten, einen olefinisch ungesättigten Substituenten aufweisenden Benzoesäureestern vorteilhaft für strahlungsempfindliche, positiv arbeitende Mischungen verwenden kann. Die daraus hergestellten Photoresists weisen eine bessere Empfindlichkeit gegenüber aktinischen Strahlen auf, lassen sich nach der Belichtung unter sehr milden Entwicklungsbedingungen, beispielsweise in einem sehr schwach alkalischen Medium, wie 1 gew.%-ige wässrige $NaHCO_3$-Lösung, entwickeln. Dabei können Schichten mit Submikron-Auflösung hergestellt werden, so dass die Photoresists auch zur Herstellung von Halbleitern verwendet werden können. Ausserdem zeigen die aus den erfindungsgemässen Photoresists hergestellten Strukturen eine hohe thermische Stabilität.

Gegenstand vorliegender Erfindung sind neue, einen olefinisch ungesättigten Substituenten aufweisende Benzoesäureester der Formel I

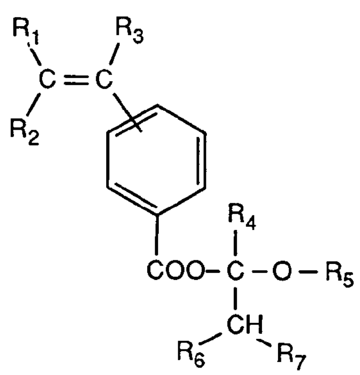

(I),

worin $R_1$ und $R_2$ unabhängig voneinander je ein Wasserstoffatom, $C_1$-$C_4$-Alkyl oder Phenyl, $R_3$ ein Wasserstoffatom, Methyl oder ein Halogenatom, $R_4$ ein Wasserstoffatom oder Methyl, $R_5$ ein $C_1$-$C_4$-Alkyl oder $C_6$-$C_{12}$-Aryl und $R_6$ und $R_7$ unabhängig voneinander je ein Wasserstoffatom, ein $C_1$-$C_4$-Alkyl oder $C_6$-$C_{12}$-Aryl bedeuten, wobei $R_5$ und $R_7$ zusammen auch einen unsubstituierten oder einen $C_1$-$C_4$-alkyl-, $C_1$-$C_4$-alkoxy-, $C_6$-$C_{12}$-aryl- oder $C_6$-$C_{12}$-aryloxysubstituierten Ethylen-, Propylen- oder Butylenrest bedeuten können.

Vorzugsweise bedeuten in der Formel I $R_1$ und $R_2$ unabhängig voneinander je ein Wasserstoffatom oder $C_1$-$C_4$-Alkyl, $R_3$ ein Wasserstoffatom oder Methyl, wobei die olefinisch ungesättigte Gruppe am Ring in para-Stellung zur Carbonyloxygruppe gebunden ist, $R_4$ ein Wasserstoffatom oder Methyl, $R_5$ ein $C_1$-$C_4$-Alkyl und $R_5$ und $R_7$ unabhängig voneinander je ein Wasserstoffatom oder ein $C_1$-$C_4$-Alkyl, wobei $R_5$ und $R_7$ zusammen auch einen unsubstituierten oder einen $C_1$-$C_4$-alkyl-, $C_1$-$C_4$-alkoxy- oder phenoxysubstituierten Ethylen-, Propylen- oder Butylenrest bedeuten können.

Insbesondere bedeuten in der Formel I $R_1$, $R_2$, $R_3$ und $R_4$ unabhängig voneinander je ein Wasserstoffatom oder Methyl, wobei die olefinisch ungesättigte Gruppe am Ring in para-Stellung zur Carbonyloxygruppe gebunden ist, $R_5$ ein Methyl und $R_5$ und $R_7$ unabhängig voneinander je ein Wasserstoffatom oder Methyl, wobei $R_5$ und $R_7$ zusammen auch einen unsubstituierten oder einen methylsubstituierten Ethylen- oder Propylenrest bedeuten können.

Bedeuten $R_1$, $R_2$, $R_5$, $R_5$ oder $R_7$ einen Alkylsubstituent, so kann dieser verzweigt oder bevorzugt geradkettig sein. Beispiele dafür sind Methyl, Ethyl, n-Propyl, Isopropyl und n-Butyl.

$R_3$ als Halogen ist vorzugsweise Chlor oder Brom.

Als $C_6$-$C_{12}$-Aryle handelt es sich hierbei um Phenyl, Biphenyl oder Naphthyl, die gegebenenfalls ein- oder mehrfach substituiert sein können, wobei als Substituenten zum Beispiel Halogenatome, vorzugsweise Chlor- oder Bromatome, oder Nitrogruppen in Betracht kommen. Geeignete substituierte Aryle sind beispielsweise o-Chlorphenyl, o-Nitrophenyl, 2,4-Dichlorphenyl und 2-Chlornaphthyl.

Als $C_1$-$C_4$-Alkoxysubstituenten kommen beispielsweise Methoxy, Ethoxy oder n-Propoxy in Frage und $C_6$-$C_{12}$-Aryloxysubstituenten sind beispielsweise Phenoxy und Naphthyloxy.

Besonders bevorzugte Verbindungen der Formel I sind 4-Vinylbenzoesäuretetrahydropyran-2-ylester und -tetrahydrofuran-2-ylester.

Die erfindungsgemässen Verbindungen der Formel I können beispielsweise hergestellt werden, indem man eine Verbindung der Formel II

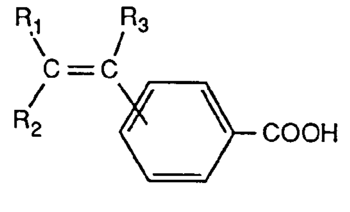

(II),

worin $R_1$, $R_2$ und $R_3$ die gleiche Bedeutung wie in Formel I haben, mit einer Verbindung der Formel III

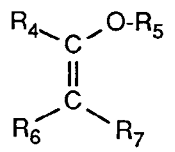

(III),

worin $R_4$, $R_5$, $R_6$ und $R_7$ die gleiche Bedeutung wie in Formel I haben, in einem sauren Medium zu einer Verbindung der Formel I umsetzt.

Die Verbindungen der Formel II sind bekannt, beispielsweise aus dem DE-Patent 3,641,099, und zum Teil im Handel erhältlich. Beispiele für Verbindungen der Formel II sind 4- oder 2-Vinylbenzoesäure, 4-(1′-Chlor)-vinylbenzoesäure, 4-Isopropenylbenzoesäure und 4-Isobutenylbenzoesäure.

Die Verbindungen der Formel III stellen ebenfalls bekannte Verbindungen dar, die zum Teil im Handel erhältlich sind. Bedeuten in Formel III $R_5$, $R_6$ und $R_7$ unabhängig voneinander je ein $C_1$-$C_4$-Akyl oder ein $C_6$-$C_{12}$-Aryl, so handelt es sich beispielsweise um Methylisobutenylether, Phenylisobutenylether, Methylstyrylether, Methylvinylether oder Ethylvinylether.

Bedeuten in Formel III $R_6$ und $R_7$ zusammen einen definitionsgemässen Alkylenrest, so handelt es sich beispielsweise um 2,3-Dihydropyran, 2,3-Dihydrofuran oder 2,3,4,5-Tetrahydrooxepin.

Bedeuten in Formel III $R_5$ und $R_7$ zusammen einen definitionsgemässen alkoxysubstituierten Alkylenrest, so kann es sich beispielsweise um 2-Methoxy-3,4-dihydropyran, das im Handel (Fluka Chemie) erhältlich ist, handeln.

Das saure Medium der Reaktionslösung kann beispielsweise dadurch hergestellt werden, indem man zur Reaktionslösung ein paar Tropfen konz. Salzsäure oder Schwefelsäure zugibt.

Die Umsetzung der Verbindungen der Formel II mit den Verbindungen der Formel III wird vorzugsweise unter einem Inertgas, insbesondere unter Stickstoff, durchgeführt, wobei leicht erhöhte Temperaturen, etwa 25 bis 80°C, angewendet werden.

Wie eingangs erwähnt, stellen die erfindungsgemässen Verbindungen der Formel I wertvolle Monomere dar, die polymerisiert, gegebenenfalls in Gegenwart von olefinisch ungesättigten Comonomeren, als strahlungsempfindliches und thermisch stabiles Resist-material eingesetzt werden können.

Gegenstand vorliegender Erfindung sind somit auch Polymere mit einem Molekulargewicht (Mw) von $10^3$ bis $10^6$, gemessen mittels Gelpermeationschromatographie, enthaltend, bezogen auf die Gesamtmenge der im Polymer vorhandenen Strukturelemente, 100 bis 5 Mol% des wiederkehrenden Strukturelementes der Formel IV

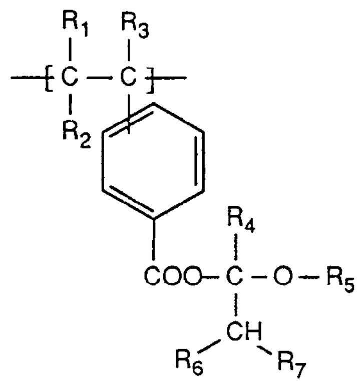

(IV),

und 95 bis 0 Mol% des wiederkehrenden Struktwelementes der Formel V

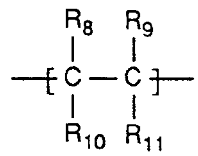

(V),

worin $R_1$, $R_2$, $R_3$, $R_4$, $R_5$, $R_6$ und $R_7$ die gleiche Bedeutung wie in Formel I haben, $R_8$ und $R_9$ unabhängig voneinander je ein Wasserstoffatom, unsubstituiertes oder durch Halogenatome, Cyano- oder Nitrogruppen substituiertes $C_1$-$C_4$-Alkyl oder ein unsubstituiertes oder durch Halogenatome, $C_1$-$C_4$-Alkoxy-, Hydroxy-, Cyano- oder Nitrogruppen substituiertes Phenyl oder Naphthyl bedeuten, und $R_{10}$ und $R_{11}$ unabhängig voneinander je ein Wasserstoff- oder Halogenatom, unsubstituiertes oder durch Halogenatome, Cyano-oder Nitrogruppen substituiertes $C_1$-$C_{12}$-Alkyl, unsubstituiertes oder durch Halogenatome, Hydroxy-, Cyano-, Nitrogruppen, $C_1$-$C_4$-Alkyl oder $C_1$-$C_4$-Alkoxy substituiertes Phenyl, Naphthyl oder Benzyl oder einen der folgenden Reste -$OR_{12}$, -$COOR_{13}$ oder -$COR_{14}$ bedeuten, wobei $R_{12}$ und $R_{13}$ unabhängig voneinander je ein Wasserstoffatom, unsubstituiertes oder durch Halogenatome, Cyano- oder Nitrogruppen substituiertes $C_1$-$C_{12}$-Alkyl, unsubstituiertes oder durch Halogen, Cyano-, Nitrogruppen, $C_1$-$C_4$-Alkyl oder $C_1$-$C_4$-Alkoxy substituiertes Phenyl oder Naphthyl bedeuten und $R_{14}$ die gleiche Bedeutung wie $R_{12}$ hat und ausserdem für den Rest

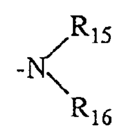

steht, worin $R_{15}$ und $R_{16}$ unabhängig voneinander die gleiche Bedeutung wie $R_{12}$ haben, oder worin $R_{10}$ und $R_{11}$ zusammen für den Rest

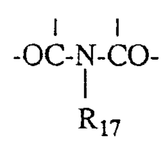

stehen, worin $R_{17}$ ein Wasserstoffatom oder ein unsubstituiertes oder ein hydroxysubstituiertes Phenyl bedeutet.

Die erfindungsgemässen Polymeren weisen vorzugsweise ein Molekulargewicht (Mw) von 5000 bis 500'000, insbesondere von 20'000 bis 150'000, auf.

Ferner enthalten die erfindungsgemässen Polymeren vorzugsweise 100 bis 20 Mol%, insbesondere 100 bis 50 Mol%, des wiederkehrenden Strukturelementes der Formel IV und 80 bis 0 Mol%, insbesondere 50 bis 0 Mol%, des wiederkehrenden Strukturelementes der Formel V.

Im wiederkehrenden Strukturelement der Formel IV haben $R_1$, $R_2$, $R_3$, $R_4$, $R_6$, $R_6$ und $R_7$ die gleiche bevor-

zugte Bedeutung wie in Formel I.

Bedeuten $R_8$, $R_9$, $R_{10}$ und $R_{11}$ im Strukturelement der Formel V ein Alkyl, so handelt es sich dabei um geradkettige oder verzweigtkettige, bevorzugt um geradkettige Alkylreste.

Halogenatome als Substituenten können Fluor, Chlor, Brom oder Jod sein und sind bevorzugt Chlor oder Brom.

Als gegebenenfalls substituiertes Alkyl kommen beispielsweise Methyl, Ethyl, 2-Chlorethyl, 2-Nitroethyl, n-Propyl, Isopropyl, n-Butyl, sek.-Butyl, tert.-Butyl, n-Pentyl, Isoamyl, n-Hexyl, 2-Ethylhexyl, n-Decyl, 6-Nitrohexyl oder 9-Bromnonyl in Frage.

Beispiele für substituiertes Phenyl oder Naphthyl sind o-, m- oder p-Chlorphenyl, o-, m-oder p-Tolyl, Xylyl, 2-Nitrophenyl oder $\alpha$-Chlornaphthyl.

Im Struktwelement der Formel V bedeuten $R_8$, $R_9$ und $R_{10}$ unabhängig voneinander vorzugsweise ein Wasserstoffatom, $C_1$-$C_6$-Alkyl oder Phenyl und bedeutet $R_{11}$ vorzugsweise ein Halogenatom, Phenyl oder Benzyl oder einen der Reste -$OR_{12}$, -$COOR_{13}$ oder -$COR_{14}$, worin $R_{12}$, $R_{13}$ und $R_{14}$ unabhängig voneinander je ein Wasserstoffatom, $C_1$-$C_6$-Alkyl oder Phenyl bedeuten und $R_{14}$ ausserdem für den Rest

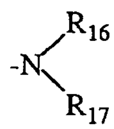

steht, worin $R_{16}$ und $R_{17}$ unabhängig voneinander die bevorzugte Bedeutung von $R_{12}$ haben.

Die erfindungsgemässen Polymeren können hergestellt werden, indem man Verbindungen der Formel I oder Gemische aus Verbindungen der Formel I und darin bis zu 95 Mol%, vorzugsweise bis zu 80 Mol%, insbesondere bis zu 50 Mol%, enthaltenen Verbindungen der Formel Va

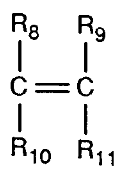

(Va),

worin $R_8$, $R_9$, $R_{10}$ und $R_{11}$ die gleiche Bedeutung wie in Formel V haben, in bekannter Weise radikalisch polymerisiert.

Die radikalische Polymerisation kann unter Anwendung verschiedener Techniken durchgeführt werden. Diese sind beispielsweise von S. Sandler und W. Karo in "Polymer Synthesis" Vol. 1-3, 1968, Academic Press, New York, beschrieben worden. Uebliche Polymerisationsverfahren sind beispielsweise Polymerisation in der Masse oder in Lösungsmitteln, Emulsions-, Suspensions- oder Fällungspolymerisation.

Die Verbindungen der Formel Va sind bekannt und zum Teil im Handel erhältlich. Neben Olefinen, wie beispielsweise Ethylen oder Propylen, sind als Beispiele für Verbindungen der Formel Va insbesondere die Vinylverbindungen zu nennen. Beispiele solcher Monomere sind die Styroltypen, wie beispielsweise Styrol, $\alpha$-Methylstyrol, p-Methylstyrol, p-Hydroxystyrol, p-Acetylstyrol oder p-Hydroxyphenylstyrol, $\alpha$,$\beta$-ungesänigte Säuren, deren Ester oder Amide, wie beispielsweise Acrylsäure, Acrylsäuremethylester, Acrylsäureamid, die entsprechenden Methacrylverbindungen, Maleinsäure, Maleinsäure-methylester, Maleinimid, p-Hydroxyphenylmaleinimide oder 4-Vinylbenzoesäure-tert.-butylester, halogenhaltige Vinylverbindungen, wie beispielsweise Vinylchlorid, Vinylfluorid, Vinylidenchlorid oder Vinylidenfluorid, Vinylester, wie beispielsweise Vinylacetat oder Vinylether, wie beispielsweise Methylvinylether oder Butylvinylether.

Weitere geeignete Verbindungen sind beispielsweise die Allylverbindungen, wie Allylchlorid, Allylbromid oder Allylcyanid.

Die Polymerisation wird in der Regel durch einen der üblichen Initiatoren der radikalischen Polymerisation eingeleitet. Dazu zählen thermische Initiatoren, wie Azoverbindungen, beispielsweise Azoisobutyronitril (AIBN), oder Peroxide, beispielsweise Benzoylperoxid, oder Redoxinitiatorsysteme, wie eine Mischung von Eisen(III)-acetyl-acetonat, Benzoin und Benzoylperoxid, oder photochemische Radikalbildner, wie Benzoin oder Benzilmethylketal.

Die Polymerisation wird bevorzugt in Lösung durchgeführt. Die Reaktionstemperatur bewegt sich im allgemeinen im Bereich von 10 bis 200°C, vorzugsweise zwischen 40 und 150°C, besonders bevorzugt zwischen 40 und 100°C.

Gegebenenfalls anwesende Lösungsmittel müssen unter den Reaktionsbedingungen inert sein. Als Lösungsmittel kommen u.a. aromatische Kohlenwasserstoffe, chlorierte Kohlenwasserstoffe, Ketone und Ether in Betracht. Beispiele dafür sind Benzol, Toluol, Xylol, Ethylbenzol, Isopropylbenzol, Ethylenchlorid, Propylenchlorid, Methylenchlorid, Chloroform, Methylethylketon, Aceton, Cyclohexanon, Diethylether oder Tetrahydrofuran.

Wie eingangs erwähnt, stellen die erfindungsgemässen Polymeren wertvolle Materialien für positive Photoresists dar, die gegenüber Säuren eine sehr gute Empfindlichkeit aufweisen und zusammen mit säuregenerierenden Photoinitiatoren ein strahlungsempfindliches Gemisch bilden. Die Empfindlichkeit der erfindungsgemässen Polymeren gegenüber Säuren wird auch in hohen Schichtdicken, beispielsweise bei 30 μm, beibehalten. Die durch Säurespaltung aus den erfindungsgemässen Polymeren erhaltenen Verbindungen sind zudem basenlöslich. Dagegen sind die erfindungsgemässen Polymeren gegenüber Basen sehr stabil, so dass im Photoresist eine sehr gute Differenzierung zwischen belichteten und unbelichteten Stellen erhalten wird.

Die Erfindung umfasst somit auch ein positiv arbeitendes strahlungsempfindliches Gemisch enthaltend, bezogen auf die Gesamtmenge der Komponenten a) und b), a) 85 bis 99 Gew.% eines Polymeren, enthaltend bezogen auf die Gesamtmenge der im Polymer vorhandenen Strukturelemente, 100 bis 5 Mol% des wiederkehrenden Strukturelementes der Formel IV und 95 bis 0 Mol% des wiederkehrenden Strukturelementes der Formel V und

b) 1 bis 15 Gew.% einer unter Einwirkung von aktinischer Strahlung säurebildenden Substanz.

Vorzugsweise enthält das strahlungsempfindliche Gemisch 90 bis 99 Gew.%, insbesondere 95 bis 98 Gew.% der Komponente a) und 1 bis 10 Gew.%, insbesondere 2 bis 5 Gew.%, der Komponente b), und die Komponente a) enthält vorzugsweise 100 bis 20 Mol%, insbesondere 100 bis 50 Mol%, des wiederkehrenden Strukturelementes der Formel IV und 80 bis 0 Mol%, insbesondere 50 bis 0 Mol%, des wiederkehrenden Strukturelementes der Formel V.

Als strahlungsempfindliche Komponenten b), die bei Lichteinwirkung Säure bilden bzw. abspalten, sind eine grosse Anzahl von Verbindungen bekannt. Dazu zählen beispielsweise Diazoniumsalze, wie sie in der Diazotypie verwendet werden, o-Chinondiazide, wie sie in bekannten positiv arbeitenden Kopiermassen verwendet werden, oder auch Halogenverbindungen, die bei Bestrahlung Halogenwasserstoffsäure bilden. Verbindungen dieses Typs sind beispielsweise in den US-PS 3,515,552, 3,536,489 oder 3,779,778, sowie in den DE-OSen 2,718,259, 2,243,621 oder 2,610, 842 beschrieben.

Als strahlungsempfindliche Komponenten b) der erfindungsgemässen Zusammensetzungen eignen sich vor allem kationische Photoinitiatoren aus der Gruppe der Iodonium- oder Sulfoniumsalze. Solche Verbindungen sind beispielsweise in "UV-Curing, Science and Technology" (Editor: S.P. Pappas, Technology Marketing Corp., 642 Westover Road, Stanford, Connecticut, USA) beschrieben.

Ferner können Sulfoxoniumsalze als strahlungsempfindliche Verbindungen verwendet werden. Solche Salze sind beispielsweise in der EP-PS 35,969 oder in der EP-A 44,274 bzw. 54,509 beschrieben. Insbesondere zu erwähnen sind aliphatische Sulfoxoniumsalze, die im tiefen UV-Bereich absorbieren.

Es lassen sich auch Verbindungen einsetzen, die bei Bestrahlung mit aktinischem Licht Sulfonsäuren freisetzen. Solche Verbindungen sind an sich bekannt und beispielsweise in der GB-A 2,120,263, den EP-A 84,515; 37,512 oder 58,638 bzw. in den US-PS 4,258,121 oder 4,371,605 beschrieben.

Werden als strahlungsempfindliche säureabspaltende Komponenten b) Salze eingesetzt, so sind diese vorzugsweise in organischen Lösungsmitteln löslich. Besonders bevorzugt handelt es sich bei diesen Salzen um Produkte mit komplexen Säuren, beispielsweise von Borfluorwasserstoffsäure, Hexafluorphosphonsäure, Hexafluorarsensäure oder Hexafluorantimonsäure.

Den erfindungsgemässen strahlungsempfindlichen Gemischen können gegebenenfalls auch Bindemittel (c) zugesetzt werden, was besonders zweckmässig ist, wenn es sich bei den lichtempfindlichen Zusammensetzungen um flüssige oder niedrigviskose Gemische handelt. In diesem Fall wirkt das erfindungsgemässe Polymer als üblicher Lösungsinhibitor. Nach dem Belichten entsteht aus dem Polymeren durch säurekatalysierte Hydrolyse eine polymere Verbindung, die basenlöslich ist.

Die Menge des Bindemittels (c) kann 30-90 Gew.%, vorzugsweise 60-90 Gew.%, betragen, bezogen auf die gesamte Menge an komponenten a), b) und c).

Die Wahl des Bindemittels erfolgt je nach dem Anwendungsgebiet und den hierfür geforderten Eigenschaften, wie Entwickelbarkeit in wässrigen und wässrig-alkalischen Lösungsmittelsystemen oder Adhäsion auf Substraten.

Geeignete Bindemittel c) sind beispielsweise Novolake, die sich von einem Aldehyd, vorzugsweise Formaldehyd, Acetaldehyd oder Furfuraldehyd, besonders jedoch von Formaldehyd, und einem Phenol ableiten. Die phenolische komponente dieser Bindemittel ist vorzugsweise Phenol selbst, oder auch halogeniertes Phenol, beispielsweise substituiert mit ein bis zwei Chloratomen, vorzugsweise p-Chlorphenol, oder sie ist ein durch ein bis zwei $C_1$-$C_9$-Alkylgruppen substituiertes Phenol, beispielsweise o- m- oder p-Kresol, ein Xylenol,

p-tert.Butylphenol oder p-Nonylphenol. Es kann sich bei der Phenolkomponente der bevorzugten Novolake aber auch um p-Phenylphenol, Resorcin, Bis-(4-hydroxyphenyl)-methan oder 2,2-Bis-(4-hydroxyphenyl)-propan handeln.

Ein Teil der phenolischen Hydroxygruppen dieser Novolake kann gegebenenfalls durch Umsetzung mit Chloressigsäure, Isocyanaten, Epoxiden oder Carbonsäureanhydriden modifiziert sein.

Weitere geeignete Bindemittel sind beispielsweise Copolymere von Maleinsäureanhydrid mit Styrol oder Vinylethern oder 1-Alkenen. Ebenfalls als Bindemittel einsetzen lassen sich:
Homo- und copolymere Acrylate und Methacrylate, z.B. Copolymere aus Methylmethacrylat/Aethylacrylat/Methacrylsäure, Poly(methacrylsäurealkylester) oder Poly(acrylsäurealkylester), wobei Alkyl = $C_1$-$C_{20}$ ist.

Vorzugsweise verwendet man als Bindemittel eine alkalilösliche Substanz, beispielsweise einen Novolak (gegebenenfalls modifiziert, wie oben beschrieben), Copolymere von Maleinsäureanhydrid mit Styrol oder Vinylethern oder 1-Alkenen, sowie Copolymere von Estern der Acrylsäure oder der Methacrylsäure mit ethylenisch ungesättigten Säuren, beispielsweise Methacrylsäure oder Acrylsäure.

Diesen alkalilöslichen Bindemitteln können gegebenenfalls noch weitere Zusatzharze beigegeben werden, wie bei den Positiv-Systemen auf Diazoketon Basis üblich. Zu diesen Zusatzharzen zählen beispielsweise Vinylpolymerisate, wie Polyvinylacetat, Polyacrylate, Polyvinylether oder Polyvinylpyrrolidone. Im allgemeinen werden jedoch nicht mehr als 20 Gew.%, bezogen auf die Menge an alkalilöslichem Bindemittel, von diesen Zusatzharzen zugefügt.

Die erfindungsgemässen Zusammensetzungen können weitere übliche Zusatzstoffe enthalten, wie z.B. Stabilisatoren, Pigmente, Farbstoffe, Füllstoffe, Haftvermittler, Verlaufmittel, Netzmittel und Weichmacher. Ferner können die Zusammensetzungen zur Applikation in geeigneten Lösungsmitteln gelöst werden.

Die erfindungsgemässen Zusammensetzungen eignen sich hervorragend als Beschichtungsmittel für Substrate aller Art, z.B. Holz, Textilien, Papier, Keramik, Glas, Kunststoffe, wie Polyester, Polyethylenterephthalat, Polyolefine oder Celluloseacetat, insbesondere in Form von Filmen, sowie Metalle, wie Al, Cu, Ni, Fe, Zn, Mg oder Co, und GaAs, Si oder $SiO_2$, bei denen durch bildmässiges Belichten eine Abbildung aufgebracht werden soll. Ein weiterer Gegenstand vorliegender Erfindung sind die beschichteten Substrate.

Die Herstellung der beschichteten Substrate kann z.B. erfolgen, indem man eine Lösung oder Suspension der Zusammensetzung herstellt.

Die Wahl des Lösungsmittels und die Konzentration richtet sich hauptsächlich nach der Art der Zusammensetzung und nach dem Beschichtungsverfahren. Die Lösung wird mittels bekannten Beschichtungsverfahren auf ein Substrat gleichförmig aufgebracht, z.B. durch Schleudern, Tauchen, Rakelbeschichtung, Vorhanggiessverfahren, Aufpinseln, Sprühen, speziell durch elektrostatisches Sprühen und Reverse-Rollbeschichtung. Es ist auch möglich, die lichtempfindliche Schicht auf einen temporären, flexiblen Träger zu bringen, und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Besonders vorteilhaft ist es, dass die erfindungsgemässen Zusammensetzungen in weiter variablen Schichtdicken eingesetzt werden können. Dieser Schichtdickenbereich umfasst Werte von ca. 0,5 µm bis mehr als 100 µm. Mit konventionellen Positiv-Systemen auf Naphthochinondiazid-Basis sind Schichtdicken von grösser als 10 µm nicht mehr verwendbar.

Mögliche Einsatzgebiete der erfindungsgemässen Zusammensetzungen sind die Verwendung als Photoresists für die Elektronik (Galvanoresist, Aetzresist, Lötstoppresist), die Herstellung von Druckplatten, wie Offsetdruckplatten oder Siebdruckformen, der Einsatz beim Formteilätzen oder der Einsatz als Mikroresist bei der Herstellung integrierter Schaltkreise.
Dementsprechend unterschiedlich sind die möglichen Schichtträger und die Verarbeitungsbedingungen der beschichteten Substrate.

Für photographische Informationsaufzeichnung dienen z.B. Folien aus Polyester, Celluloseacetat oder mit kunststoff beschichtete Papiere; für Offsetdruckformen speziell behandeltes Aluminium, für die Herstellung gedruckter Schaltungen kupferkaschierte Laminate und für die Herstellung von integrierten Schaltkreisen Siliziumwafer. Die Schichtdicken für photographische Materialien und Offsetdruckformen betragen ca. 0,5 µm bis 10 µm; für gedruckte Schaltungen 1 bis ca. 100 µm.

Nach dem Beschichten wird das Lösungsmittel in der Regel durch Trocknen entfernt und es resultiert eine Schicht des Photoresists auf dem Träger.

Nach der in üblicher Weise erfolgten bildmässigen Belichtung des Materials werden die belichteten Stellen des Photolackes durch Herauslösen in einem Entwickler entfernt.

Die Wahl des jeweiligen Entwicklers richtet sich nach der Art des Photolackes, insbesondere nach der Natur des verwendeten Bindemittels oder der entstehenden Photolyseprodukte. Der Entwickler kann wässrige Lösungen von Basen umfasssen, denen gegebenenfalls organische Lösungsmittel oder deren Mischungen

zugesetzt wurden.

Besonders bevorzugt als Entwickler werden wässrig alkalische Lösungen, wie sie auch für die Entwicklung von Naphthochinondiazidschichten eingesetzt werden. Dazu zählen insbesondere wässrige Lösungen von Alkalimetallsilikaten, -phosphaten, -hydroxiden und -carbonaten. Diesen Lösungen können gegebenenfalls noch kleinere Mengen an Netzmitteln und/oder organischen Lösungsmitteln zugesetzt sein.

Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.

Der Begriff 'bildmässige' Belichtung beinhaltet sowohl die Belichtung durch eine Photomaske, die ein vorbestimmtes Muster enthält, beispielsweise ein Diapositiv, die Belichtung durch einen Laserstrahl, der beispielsweise computergesteuert über die Oberfläche des beschichteten Substrates bewegt wird, und auf diese Weise ein Bild erzeugt, sowie die Bestrahlung mit computergesteuerten Elektronenstrahlen.

Die Lichtempfindlichkeit der erfindungsgemässen Zusammensetzungen reicht in der Regel vom UV-Gebiet (ca. 200 nm) bis ca. 600 nm und umspannt somit einen sehr breiten Bereich. Als Lichtquellen kommen daher eine grosse Anzahl der verschiedensten Typen zur Anwendung. Es sind sowohl Punktlichtquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen, photographische Flutlichtlampen, Elektronenstrahlen und Röntgenstrahlen. Der Abstand zwischen Lampe und erfindungsgemässem Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser. Bei dieser Art der Belichtung ist eine Photomaske im Kontakt mit der Photopolymerschicht nicht mehr unbedingt nötig; der gesteuerte Laser-Strahl schreibt direkt auf die Schicht. Hier ist die hohe Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die Erfindung betrifft daher auch die unter Verwendung besagter Zusammensetzungen hergestellten Druckformen, gedruckten Schaltungen, integrierten Schaltkreise oder silberlosen photographischen Filme.

Beispiel 1: Herstellung von 4-Vinylbenzoesäuretetrahydropyran-2-ylester

20 g (135 mMol) 4-Vinylbenzoesäure und 22,7 g (270 mMol) 3,4-Dihydro-2H-pyran werden in einem 100 ml Rundkolben mit Magnetrührer vorgelegt. Zur Suspension gibt man 4 Tropfen konz. Salzsäure. Unter Stickstoff wird das Reaktionsgemisch bei 40°C gerührt. Nach etwa 40 Minuten erhält man eine klare Lösung, die noch 40 Minuten weitergerührt wird. Die Lösung wird auf eiskalte 2n Natriumhydroxidlösung gegossen und zweimal mit Diethylether ausgezogen. Die Etherphase wird mit Wasser gewaschen und mit Natriumsulfat getrocknet. Nach dem Abdampfen des Ethers am Rotationsverdampfer wird die erhaltene Flüssigkeit in n-Hexan gelöst und mit Aktivkohle behandelt, filtriert und das Lösungsmittel am Rotationsverdampfer entfernt. Die klare, farblose Flüssigkeit wird anschliessend am Hochvakuum getrocknet.
Ausbeute: 27,5 g (88 % der Theorie). Die Substanz ist nicht destillierbar.
Elementaranalyse (Mikroanalyse):

| | berechnet | gefunden |
|---|---|---|
| C = | 72,39 % | 72,41 % |
| H = | 6,94 % | 7,23 % |

Das $^1$H-NMR-Spektrum ($CDCl_3$) stimmt mit der Struktur für 4-Vinylbenzoesäuretetrahydropyran-2-ylester überein.

Beispiel 2: Herstellung von 4-Isopropenylbenzoesäuretetrahydropyran-2-ylester

5 g 4-Isopropenylbenzoesäure (31 mMol) und 5,2 g 3,4-Dihydro-2H-pyran (62 mMol) werden in einem 25 ml Rundkolben mit Magnetrührer vorgelegt. Zur Suspension gibt man 2 Tropfen konz. Salzsäure. Unter Stickstoff wir das Reaktionsgemisch bei 40°C gerührt. Innerhalb 20 Minuten geht die Suspension in eine Lösung über. Nach 60 Minuten wird die Lösung auf eiskalte 2n NaOH-Lösung gegossen und mit 50 ml Ether ausgezogen. Die organische Phase wird mit Natriumchlorid gesättigtem Wasser gewaschen, mit $Na_2SO_4$ getrocknet,

filtriert und eingedampft.
Die leicht bräunliche Flüssigkeit wird über Kieselgel/Chloroform chromatographiert.
Ausbeute: 2,7 g (36 % der Theorie).
Mikroanalyse:

| | berechnet | gefunden |
|---|---|---|
| C = | 73,15 % | 73,20 % |
| H = | 7,37 % | 7,59 % |

Das $^1$H-NMR-Spektrum ($CDCl_3$) stimmt mit der Struktur für 4-Isopropenylbenzoesäuretetrahydropyran-2-ylester überein.

Beispiel 3: Herstellung von 4-Vinylbenzoesäuretetrahydrofuran-2-ylester

24 g (162 mMol) 4-Vinylbenzoesäure und 28,4 g (405 mMol) 2,3-Dihydrofuran werden in einem 100 ml Rundkolben mit Magnetrührer vorgelegt. Zur Suspension gibt man 3 Tropfen konz. Salzsäure. Unter Stickstoff wird das Reaktionsgemisch bei 40°C gerührt. Die Suspension geht innerhalb einer Minute in eine klare Lösung über.
Nach 30 Minuten wird die Lösung auf eiskalte 1n NaOH-Lösung gegossen. Es wird zweimal mit Diethylether ausgezogen und die vereinigte Etherphase mit Wasser gewaschen, mit Natriumsulfat getrocknet und der Ether am Rotationsverdampfer abgetrieben.
Man erhält eine klare Flüssigkeit, die bei 150°C/0,03 mbar siedet.
Ausbeute: 28,6 g (81 % der Theorie).
Mikroanalyse:

| | berechnet | gefunden |
|---|---|---|
| C = | 71,54 % | 71,51 % |
| H = | 6,47 % | 6,60 % |

Das $^1$H-NMR-Spektrum ($CDCl_3$) stimmt mit der Struktur für 4-Vinylbenzoesäuretetrahydrofuran-2-ylester überein.

Beispiel 4: Herstellung von 4-Vinylbenzoesäure-1-methoxyisobutylester

Es wird 1-Methoxyisobuten nach der Vorschrift von H. Böhme und H. Bentler, beschrieben in Chem. Ber. 1959, 89, 1464-1468, hergestellt. Man erhält eine Flüssigkeit, die bei Normaldruck über eine Füllkörperkolonne destilliert wird und einen Siedepunkt von 70°C besitzt. Gaschromatographische Analysen zeigen eine Reinheit des Produktes von 98 %.
Man erreicht eine Ausbeute von 30 %.
Mikroanalyse:

| | berechnet | gefunden |
|---|---|---|
| C = | 69,7 % | 68,6 % |
| H = | 11,7 % | 11,2 % |

Das $^1$H-NMR-Spektrum ($CDCl_3$) stimmt mit der Struktur für 1-Methoxyisobuten überein.
15 g (100 mMol) 4-Vinylbenzoesäure werden in 17,2 g (200 mMol) 1-Methoxyisobuten suspendiert. Man gibt 5 Tropfen konz. Salzsäure dazu und rührt während 7 Stunden bei 40°C. Es entsteht eine klare Lösung, die mit Diethylether verdünnt wird und zweimal mit wässriger $NaHCO_3$ ausgewaschen wird. Die Etherphase wird mit $Na_2SO_4$ getrocknet, dann filtriert und eingedampft. Der Rückstand wird bei 125°C und 0,065 mbar destilliert. Man erhält 6 g 4-Vinylbenzoesäure-1-methoxyisobutylester als klare Flüssigkeit.
Mikroanalyse:

| | berechnet | gefunden |
|---|---|---|
| C = | 71,7 % | 71,1 % |
| H = | 7,7 % | 7,9 % |

Das $^1$H-NMR-Spektrum ($CDCl_3$) stimmt mit der Struktur für 4-Vinylbenzoesäure-1-methoxyisobutylester überein.

Beispiel 5: Herstellung von Poly-(4-vinylbenzoesäuretetrahydropyran-2-ylester)

8 g (34 mMol) 4-Vinylbenzoesäuretetrahydropyran-2-ylester, 57 mg (1 Mol%) Azoisobutyronitril (AIBN) und 32 ml Toluol werden in eine 50 ml Ampulle mit Magnetrührer gegeben. Bei -78°C wird zweimal entgast und die Ampulle im Hochvakuum zugeschmolzen. Bei 70°C wird 12 Stunden lang polymerisiert. Die viskose Lösung wird auf Methanol gegossen wobei das Polymer ausfällt. Das Polymer wird abgetrennt, getrocknet und in 60 ml Methylenchlorid gelöst. Diese Lösung wird zu 800 ml Methanol getropft und das ausgefällte Polymer abgetrennt. Das weisse Polymer wird am Hochvakuum bei 40°C getrocknet.
Ausbeute: 5,8 g (72 % der Theorie).
Gelpermeationschromatographische Bestimmung des Molekulargewichtes (GPC) in Tetrahydrofuran (THF): Mw: 63'000 Mn: 34'000 Mw/Mn = 1,85.
Mikroanalyse:

| | berechnet | gefunden |
|---|---|---|
| C = | 72,39 % | 72,24 % |
| H = | 6,94 % | 7,10 % |

Beispiel 6: Homopolymerisation von 4-Vinylbenzoesäuretetrahydrofuran-2-ylester

10 g (46 mMol) 4-Vinylbenzoesäuretetrahydrofuran-2-ylester, 75 mg (1 Mol%) AIBN und 43 ml Toluol werden in einem 100 ml Rundkolben mit Magnetrührer vorgelegt. Bei -78°C wird die Lösung zweimal entgast und unter Argon gesetzt.
Bei 70°C lässt man 16 Stunden polymerisieren. Die viskose Flüssigkeit wird auf n-Hexan getropft, wobei ein weisses Polymer ausfällt. Das Polymer wird abgetrennt, getrocknet und in 100 ml Methylenchlorid gelöst. Diese Lösung wird auf 1,3 Liter n-Hexan getropft, das Polymer abgetrennt und bei 0,03 mbar/40°C getrocknet.
Ausbeute: 4,6 g (46 % der Theorie).
GPC (THF): Mw: 37'000 Mn: 11'000 Mw/Mn = 3,36.
Mikroanalyse:

| | berechnet | gefunden |
|---|---|---|
| C = | 71,54 % | 71,24 % |
| H = | 6,47 % | 6,52 % |

Applikationsbeispiele

Beispiel A

4 g Poly-(4-vinylbenzoesäuretetrahydropyran-2-ylester) werden mit 0,2 g Thiophenoxyphenyldiphenylsulfoniumhexafluoroarsenat in 40 g Cyclopentanon gelöst. Diese Lösung wird durch ein 0,5 Mikronfilter filtriert und auf eine Siliziumscheibe aufgetragen. Durch Schleuderbeschichtung mit 2500 U/min wird ein homogener Film auf der Siliziumscheibe erzeugt. Der Film wird bei 90°C während 2 Minuten getrocknet. Die Filmdicke beträgt 1,2 Mikron.
Der Film wird durch eine Maske und ein Engbandfilter mit Licht der Wellenlänge 308 nm belichtet. Dabei wird eine Dosis von 4-5 mJ/cm$^2$ benötigt.
Nach der Belichtung wird der Resistfilm auf einer Heizplatte 1 Minute lang bei 110°C erwärmt und anschliessend in 0,5 %-iger wässriger $NaHCO_3$-Lösung bei 22°C während 2 Minuten entwickelt. Die belichteten Zonen werden dabei herausgelöst, der Resist ist also positiv arbeitend. Der Resistfilm wird mit deionisiertem Wasser gespült und bei 90°C getrocknet.
Analysen im Rasterelektronenmikroskop zeigen, dass der Resist 0,5 Mikron Strukturen auflöst und die Flankensteilheit der Strukturen fast 90° beträgt.

Beispiel B

4 g Poly(4-vinylbenzoesäuretetrahydrofuran-2-ylester) werden mit 0,2 g Thiophenoxyphenyldiphenylsulfoniumhexafluoroarsenat in 20 g Cyclopentanon gelöst und durch ein 0,5 Mikron-Filter auf eine Siliziumscheibe aufgebracht. Bei 2500 U/min wird mittels Schleuderbeschichtung ein homogener Film auf der Siliziumscheibe erzeugt. Der Film wird bei 90°C während 2 Minuten getrocknet. Die Filmdicke beträgt 1 Mikron. Analog Beispiel A wird der Film belichtet und entwickelt. Eine Belichtungsdosis von 4 $mJ/cm^2$ bei 308 nm genügt, um Submikron-Strukturen aufzulösen.

## Patentansprüche

1. Einen olefinisch ungesättigten Substituenten aufweisende Benzoesäureester der Formel I

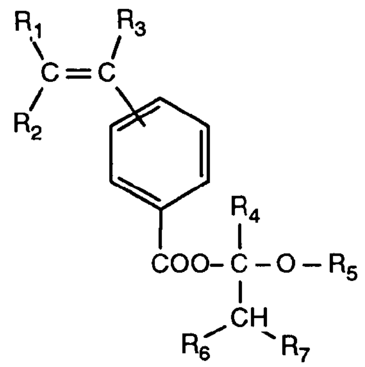

(I),

worin $R_1$ und $R_2$ unabhängig voneinander je ein Wasserstoffatom, $C_1$-$C_4$-Alkyl oder Phenyl, $R_3$ ein Wasserstoffatom, Methyl oder ein Halogenatom, $R_4$ ein Wasserstoffatom oder Methyl, $R_5$ ein $C_1$-$C_4$-Alkyl oder $C_6$-$C_{12}$-Aryl und $R_6$ und $R_7$ unabhängig voneinander je ein Wasserstoffatom, ein $C_1$-$C_4$-Alkyl oder $C_6$-$C_{12}$-Aryl bedeuten, wobei $R_5$ und $R_7$ zusammen auch einen unsubstituierten oder einen $C_1$-$C_4$-alkyl-, $C_1$-$C_4$-alkoxy-, $C_6$-$C_{12}$-aryl- oder $C_6$-$C_{12}$-aryloxysubstituierten Ethylen-, Propylen- oder Butylenrest bedeuten können.

2. Benzoesäureester gemäss Anspruch 1, worin in der Formel I $R_1$ und $R_2$ unabhängig voneinander je ein Wasserstoffatom oder $C_1$-$C_4$-Alkyl bedeuten und $R_3$ für ein Wasserstoffatom oder Methyl steht, wobei die olefinisch ungesättigte Gruppe am Ring in para-Stellung zur Carbonyloxygruppe gebunden ist, $R_4$ ein Wasserstoffatom oder Methyl, $R_5$ ein $C_1$-$C_4$-Alkyl und $R_6$ und $R_7$ unabhängig voneinander je ein Wasserstoffatom oder ein $C_1$-$C_4$-Alkyl bedeuten, wobei $R_5$ und $R_7$ zusammen auch einen unsubstituierten oder einen $C_1$-$C_4$-alkyl-, $C_1$-$C_4$-alkoxy- oder phenoxysubstituierten Ethylen-, Propylen- oder Butylenrest bedeuten können.

3. Benzoesäureester gemäss Anspruch 1, worin in der Formel I $R_1$, $R_2$, $R_3$ und $R_4$ unabhängig voneinander je ein Wasserstoffatom oder Methyl bedeuten, wobei die olefinisch ungesättigte Gruppe am Ring in para-Stellung zur Carbonyloxygruppe gebunden ist, $R_5$ ein Methyl und $R_5$ und $R_7$ unabhängig voneinander je ein Wasserstoffatom oder Methyl bedeuten, wobei $R_5$ und $R_7$ zusammen auch einen unsubstituierten oder einen methylsubstituierten Ethylen- oder Propylenrest bedeuten können.

4. 4-Vinylbenzoesäuretetrahydropyran-2-ylester und -tetrahydrofuran-2-ylester, 4-Isopropenylbenzoesäuretetrahydropyran-2-ylester und 4-Vinylbenzoesäure-1-methoxyisobutylester als Verbindungen der Formel I gemäss Anspruch 1.

5. Verfahren zur Herstellung von Verbindungen der Formel I gemäss Anspruch 1, dadurch gekennzeichnet, dass man eine Verbindunb der Formel II

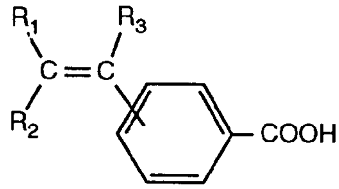

(II),

worin $R_1$, $R_2$ und $R_3$ die gleiche Bedeutung wie in Formel I haben, mit einer Verbindung der Formel III

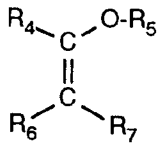

(III),

worin $R_4$, $R_5$, $R_5$ und $R_7$ die gleiche Bedeutung wie in Formel I haben, in einem sauren Medium zu einer Verbindunb der Formel I umsetzt.

6. Polymere mit einem Molekulargewicht (Mw) von $10^3$ bis $10^6$, gemessen mittels Gelpermeationschromatographie, enthaltend, bezogen auf die Gesamtmenbe der im Polymer vorhandenen Strukturelemente, 100 bis 5 Mol% des wiederkehrenden Strukturelementes der Formel IV

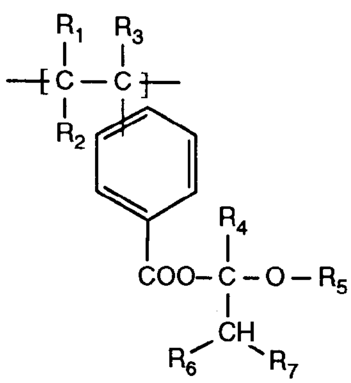

(IV),

und 95 bis 0 Mol% des wiederkehrenden Strukturelementes der Formel V

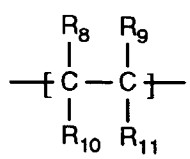

(V),

worin $R_1$, $R_2$, $R_3$, $R_4$, $R_5$, $R_5$ und $R_7$ die gleiche Bedeutung wie in Formel I gemäss Anspruch 1 haben, $R_5$ und $R_9$ unabhängig voneinander je ein Wasserstoffatom, unsubstituiertes oder durch Halogenatome, Cyano- oder Nitrogruppen substituiertes $C_1$--$C_4$-Alkyl oder ein unsubstituiertes oder durch Halogenatome, $C_1$-$C_4$-Alkoxy-, Hydroxy-, Cyano-oder Nitrogruppen substituiertes Phenyl oder Naphthyl bedeuten, und $R_{10}$ und $R_{11}$ unabhängig voneinander je ein Wasserstoff- oder Halogenatom, unsubstituiertes oder durch Halogenatome, Cyano- oder Nitrogruppen substituiertes $C_1$-$C_{12}$-Alkyl, unsubstituiertes oder durch Halogenatome, Hydroxy-, Cyano-, Nitrogruppen, $C_1$-$C_4$-Alkyl oder $C_1$-$C_4$-Alkoxy substituiertes Phenyl, Naphthyl oder Benzyl oder einen der folgenden Reste $-OR_{12}$, $-COOR_{13}$ oder $-COR_{14}$ bedeuten, wobei $R_{12}$ und $R_{13}$ unabhängig voneinander je ein Wasserstoffatom, unsubstituiertes oder durch Halogenatome, Cyano- oder Nitrogruppen substituiertes $C_1$-$C_{12}$-Alkyl, unsubstituiertes oder durch Halogen, Cyano-, Nitrogruppen, $C_1$-

$C_4$-Alkyl oder $C_1$-$C_4$-Alkoxy substituiertes Phenyl oder Naphthyl bedeuten und $R_{14}$ die gleiche Bedeutung wie $R_{12}$ hat und ausserdem für den Rest

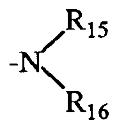

steht, worin $R_{15}$ und $R_{16}$ unabhängig voneinander die gleiche Bedeutung wie $R_{12}$ haben.

7. Polymere gemäss Anspruch 6, enthaltend 100 bis 20 Mol% des wiederkehrenden Strukturelementes der Formel IV und 80 bis 0 Mol% des wiederkehrenden Strukturelementes der Formel V.

8. Strahlungsempfindliches Gemisch enthaltend, bezogen auf die Gesamtmenge der Komponenten a) und b),
   a) 85 bis 99 Gew.% eines Polymeren gemäss Anspruch 6 enthaltend, bezogen auf die Gesamtmenge der im Polymer vorhandenen Strukturelemente, 100 bis 5 Mol% des wiederkehrenden Strukturelementes der Formel IV und 95 bis 0 Mol% des wiederkehrenden Strukturelementes der Formel V und
   b) 1 bis 15 Gew.% einer unter Einwirkung von aktinischer Strahlung säurebildenden Substanz.

9. Gemisch gemäss Anspruch 8, enthaltend
   a) 90 bis 99 Gew.% eines Polymeren, enthaltend 100 bis 20 Mol% des wiederkehrenden Strukturelementes der Formel IV und 80 bis 0 Mol% des wiederkehrenden Strukturelementes der Formel V und
   b) 1 bis 10 Gew.% einer unter Einwirkunb von aktinischer Strahlung säurebildenden Substanz.

10. Die unter Verwendung der Zusammensetzung gemäss Anspruch 8 hergestellten Druckformen, gedruckten Schaltungen, integrierten Schaltkreise oder silberlosen photographischen Filme.